# FASCICULE DE BREVET EUROPEEN

(11) **EP 0 873 748 B1**
(45) Date de publication et mention de la délivrance du brevet: **06.11.2002**
(21) Numéro de dépôt: 98400976.1
(22) Date de dépôt: 22.04.1998
(51) Int. Cl.: A61K 7/48, A61K 7/043

(54) **Composition cosmétique aqueuse formant par séchage un film démaquillable à l'eau**
Wässrige kosmetische Zubereitung, welche durch Trocknung einen mit Wasser abschminkbaren Film bildet
Aqueous cosmetic composition forming by drying a water removable film

(30) Priorité: 23.04.1997 FR 9705013
(43) Date de publication de la demande: 28.10.1998
(73) Titulaire: Fiabila, 28130 Maintenon (FR)
(72) Inventeur: Malnou, Alain, 27350 Routot (FR)
(74) Mandataire: Loyer, Bertrand

(56) Documents cités:
- EP-A- 0 628 304
- FR-A- 2 722 404
- FR-A- 2 733 147
- BASE DE DONN ES: "CHEMICAL ABSTRACTS" (SERVEUR: STN); Abrégé 125:17698, Colombus OH, USA; & JP 08 157 329(MITSUBISHI PEN. CO.) 18 JUIN 1996. XP002051702

## Description

La présente invention concerne d'une manière générale une composition cosmétique.

De manière connue en soi, il existe sur le marché de très nombreux types de compositions cosmétiques permettant le maquillage et/ou le soin.

Ces compositions sont par exemple des crèmes ou des fards que l'on étale sur la peau et qui sont enlevées par le simple effet d'un frottement non volontaire tel que celui des habits, soit par un frottement volontaire à l'aide d'un coton. Ce type de composition disparaît peu à peu et nécessite des applications répétées.

Un autre type de composition largement utilisé consiste en un vernis qui sèche en formant un film protecteur. Ces vernis ne disparaissent pratiquement pas d'eux-mêmes et nécessitent un démaquillage spécial au moyen de solvants plus ou moins dangereux à manipuler du fait de leur volatilité.

Il existe également des compositions formant un film lors de leur séchage, ledit film étant nettoyable à l'eau, c'est-à-dire pouvant être démaquillé en étant dissout dans l'eau. Ces films sont actuellement obtenus en utilisant des polymères en solution dans l'eau ou en solution hydro-alcoolique, des dispersions aqueuses de polymères partiellement neutralisées ou des émulsions aqueuses alcali-solubles. Ces compositions présentent toutes des inconvénients : les polymères en solution posent des problèmes de viscosité et de séchage, les dispersions de polymères partiellement neutralisées nécessitent un passage par une phase au cours de laquelle les polymères sont en solution dans un solvant organique, une partie de ce solvant pouvant être retenue dans la dispersion, ces dispersions posent également des problèmes de stabilité pigmentaire, les émulsions alcali-solubles sont peu solubles dans l'eau lorsqu'elles ont séché et leur démaquillage nécessite une solution basique.

La demanderesse a donc cherché à proposer une composition cosmétique ne présentant pas ces inconvénients.

A cet effet, l'invention concerne une composition cosmétique aqueuse formant par séchage un film démaquillable à l'eau, caractérisée en ce qu'elle contient une dispersion de copolymères styrène acrylique fabriquée par polymérisation en émulsion dans l'eau et neutralisation à l'aide d'une base peu volatile et en ce que ladite dispersion a une teneur en solides d'environ 46 % en poids et est présente en quantité telle que le taux de solide dans la composition est compris entre 20 et 40 % en poids.

La composition est encore remarquable en ce que :
- l'indice d'acide de ladite dispersion est inférieur à 80 mg de KOH par gramme de polymère, et de préférence inférieur à 50 mg de KOH par gramme de polymère,
- la dispersion mise en oeuvre est une émulsion de copolymères styrène acrylique ayant un température de transition vitreuse élevée, neutralisée à l'aide d'une base minérale, telle que de la soude ou de la potasse, ou d'une base organique très lourde comme une amine lourde, triéthanolamine ou tris(hydroxyméthyl)aminométhane par exemple,
- sa température minimale de formation de film est adaptée à sa destination par l'ajout d'un coalescent et/ou un plastifiant qui favorise la formation du film,
- le coalescent est choisi parmi ceux ayant une température d'ébullition supérieure à 250 °C,
- sa sensibilité à l'eau est adaptée par l'ajout d'un tensioactif du type éther polyoxyéthyléné d'alcool gras,
- elle comporte une émulsion présentant une température de transition vitreuse et une température de formation de film très basses,
- sa resolubilisation à la goutte après séchage et coalescence est effectuée en moins de 30 secondes,
- elle comprend des additifs tels que des agents de conservation, des produits siliconés, des épaississants minéraux ou organiques, des agents mouillants, des additifs d'amertume, des colorants, des parfums, etc...
- elle est résistante aux solvants du type acétates de butyle et d'éthyle utilisés dans les vernis classiques ce qui permet de l'utiliser pour réaliser des motifs décoratifs sur les ongles, lesdits motifs étant recouverts d'une couche de vernis classique.

La dispersion de polymère utilisée dans cette composition est un latex qui forme un film au cours de son séchage, ledit film étant soluble à l'eau même après un long temps de séchage ce qui permet un démaquillage à l'eau très facilement réalisable et non agressif. On dit également que le film est nettoyable à l'eau.

La composition suivant l'invention peut être colorée pour le maquillage et être par exemple utilisée comme vernis à ongles, vernis lèvres ou mascaras.

Elle peut également être additionnée d'un parfum, d'un goût, par exemple un goût amer pour empêcher de sucer le pouce ou de ronger les ongles, ou d'un produit de soin tout en étant ou non colorée. La réalisation d'un produit de soin à l'aide d'une composition suivant l'invention permet d'assurer que le produit reste en contact avec la zone à traiter tant que le film créé lors du séchage n'est pas retiré volontairement par l'utilisateur. Il est ainsi possible d'appliquer un produit de soin avant de se coucher et de l'enlever sous la douche au réveil, le produit ayant agit toute la nuit sans avoir été absorbé par la literie comme c'est généralement le cas.

La composition cosmétique suivant l'invention peut bien entendu être colorée et/ou parfumée et/ou additionnée d'un produit de soin.

Le latex est réalisé sans apport de solvants organiques ce qui permet de fournir une composition cosmétique plus saine et non inflammable. Une telle composition ne déshydratera pas les ongles et pourra être utilisée sans danger même par des enfants.

Ce latex est par exemple une émulsion de copolymères styrène acrylique ayant un température de transition vitreuse (Tg) élevée. Il est neutralisé à l'aide d'une base minérale, telle que de la soude ou de la potasse, ou d'une base organique très lourde comme une amine lourde, triéthanolamine ou tris(hydroxyméthyl)aminométhane par exemple, ce qui lui confère un pH de l'ordre de 8.

La viscosité de ces latex est généralement peu élevée, de l'ordre de 200 à 500 mPa.s, ce qui présente l'avantage de réduire le temps de séchage du fait que les volumes d'eau à évaporer sont faibles.

Le taux de solide des émulsions utilisées dans la composition suivant l'invention est de l'ordre de 46 %, ces émulsions seront utilisées dans les compositions en quantité telle que le taux de solide de l'émulsion dans les compositions soit compris entre 20 et 40 %.

Un autre avantage de ces latex tient au fait qu'ils ont un indice d'acide faible, inférieur à 80 mg de KOH par gramme de polymère, et de préférence inférieur à 50 mg de KOH par gramme de polymère.

Pour obtenir une composition cosmétique colorée dont la couleur à l'état liquide est la même que celle du film obtenu après séchage, on choisit une dispersion de polymères dont les particules ont une taille inférieure à 100 nanomètres.

Ces émulsions ont une température minimale de formation de film (TMFF) élevée ce qui permet d'adapter celle de la composition cosmétique les comportant en fonction de la destination de celle-ci en ajoutant un coalescent et/ou un plastifiant qui favorise la formation du film.

Le coalescent est choisi parmi ceux ayant une température d'ébullition (Eb) supérieure à 250 °C afin de ne pas être un composé organique volatil risquant de s'évaporer au cours de la vie du film et de remplir ainsi sa mission de plastifiant en apportant une certaine souplesse au film, on utilise par exemple un tripropylèneglycol n-butyl éther (TPnB).

De manière à adapter la sensibilité à l'eau de la composition cosmétique suivant l'invention à sa destination, on ajoute un tensioactif du type éther polyoxyéthyléné d'alcool gras. Ce tensioactif non-ionique présente l'avantage d'améliorer à la fois la coalescence, la souplesse et la faculté de démaquillage à l'eau.

Ce tensioactif est ajouté dans une proportion allant de 0 à 8 %, de préférence de 3 à 6 %.

Lorsque, du fait de la destination de la composition cosmétique, il est nécessaire de diminuer sa solubilité à l'eau ou d'avoir une meilleure coalescence, on ajoute une émulsion présentant une température de transition vitreuse et une température de formation de film très basses du type de celles connues pour diminuer ce que l'on appelle usuellement le "tack" du film c'est-à-dire la capacité du film à rester collant.

Cette seconde émulsion est utilisée dans une proportion comprise entre 0 et 20 %, compté en solide.

Afin de colorer les compositions cosmétiques selon l'invention, on utilise des pigments usuellement mis en oeuvre en cosmétique en les dispersant dans la dispersion à l'aide d'un agent mouillant polymérique soluble dans l'eau. On choisit de préférence des pigments insolubles dans l'eau et stables aux pH.

Des hectorites synthétiques sont mises en oeuvre pour maintenir les pigments en suspension, spécialement lors de l'utilisation de nacres. Ces hectorites sont ajoutées dans une proportion de 0,3 à 1 % en poids du mélange.

Un épaississant uréthanne et un épaississant acryliques sont utilisés lorsqu'il est nécessaire d'adapter la viscosité d'application et la viscosité à bas gradient de la composition cosmétique. Un tel mélange permet d'obtenir une bonne rhéologie d'application et de ne pas subir de synérèse au stockage, c'est-à-dire de ne pas se séparer en deux phases, incolore ou laiteuse et colorée.

Il est également possible d'améliorer la glissance en ajoutant un additif siliconé.

L'addition d'un mélange de parabens solubilisés dans les coalescents permet de protéger la composition cosmétique des bactéries.

Les tests de solubilité sont réalisés en appliquant une couche de 100 microns du produit humide sur une plaque d'aluminium et en faisant sécher soit à température ambiante pendant 1, 3, 6 ou 24 heures, soit sur une plaque chauffante à 30°C.

L'on dépose ensuite une goutte d'eau toutes les 5 secondes sur le film avant d'essuyer avec un papier doux, et l'on note le temps à partir duquel la plaque est nettoyée, le film étant totalement enlevé. Ce temps est généralement de 15 à 25 secondes après 24 heures de séchage à température ambiante, on considère qu'une resolubilisation à la goutte après séchage et coalescence en moins de 30 secondes est un bon résultat.

Ce test peut être complété par un jugement qualitatif en frottant le film avec un papier ou un coton imbibé d'eau.

Nous allons donner maintenant quelques exemples de formulation de compositions cosmétiques suivant l'invention.

La formule type pour un vernis à ongle est la suivante :

| Emulsion neutralisée avec une base non volatile : 50 à 90 | |
|---|---|
| Phénoxy éthanol ou phénoxy propanol | 0 à 1 |
| Méthyl paraben | 0 à 0,5 |
| Propyl paraben | 0 à 0,2 |
| TPnB ou coalescent Eb>250°C | 2 à 8 |
| Ether polyoxyéthyléné d'alcool gras | 2 à 8 |
| Emulsion basse Tg | 0 à 40 |
| Hectorite à 10 % dans l'eau | 0 à 7 |
| Epaississant uréthanne | 0 à 0,5 |
| Silicone | 0 à 1 |
| Epaississant acrylique à 33 % dans l'eau | 0,5 à 3 |
| Parfum | 0 à 0,8 |
| Dispersions pigmentaires | 0 à 10 |
| Eau en quantité suffisante pour régler la viscosité de la composition | |

Les quantités données sont exprimées en pourcentage en poids dans la composition.

Un vernis incolore peut à titre d'exemple être fabriqué de la manière suivante.

On charge dans un récipient muni d'une agitation 65 parties en poids de Dypol 2160 (nom commercial d'une émulsion neutralisée avec une base non volatile commercialisée par Dyflex Polymer).

On prépare une solution en mélangeant 6 parties de EPH (phénoxy éthanol), 50 parties de TPnB, 6 parties de m Paraben, 2 parties de p Paraben et 36 parties de Volpo N3 (nom commercial d'un éther polyoxyéthyléné d'alcool gras commercialisée par Croda).

On ajoute goutte à goutte 8 parties de ce mélange dans le récipient et on agite 15 minutes.

On ajoute ensuite sous agitation 25 parties de Dyflex LP 9334 Tg=-17°C (nom commercial d'une émulsion à basse Tg commercialisée par Dyflex Polymer), puis 5 parties de Laponite XLS à 10 % dans l'eau (nom commercial d'une hectorite commercialisée par Laporte), 0,6 parties de Fluide 70646 (nom commercial d'un silicone commercialisé par Rhône Poulenc), 0,5 parties de Acrysol A44 (nom commercial d'un épaississant uréthanne commercialisé par Rohm & Haas) et 1,5 parties de Viscoatex 538C 33 % dans l'eau (nom commercial d'un épaississant acrylique commercialisé par Coatex).

Un tel mélange permet d'obtenir une composition ayant une viscosité brookfield mobile de 910 cps à 3 à 6 tours/mn et de 800 cps à 60 tours/mn au Brookfield mobile 3.

Pour fabriquer une pâte à teinter rouge, on mélange dans un récipient muni d'une agitation rapide 50 parties en poids de Joncryl 8250 (nom commercial d'une émulsion neutralisée avec une base non volatile commercialisée par Johnson), 22,5 parties d'eau, 2 parties de Disperbyk 190 (nom commercial d'un agent mouillant commercialisé par Byk Chemie), 0,5 parties de Byk 019 anti mousse (nom commercial de Byk Chemie).

On agite pendant 5 minutes puis on charge sous agitation 20 parties de pigment Cl 12490.

On introduit ensuite des billes de broyage dans le récipient et on broie pendant 1 heure.

On ajoute 5 parties d'eau et on retire ensuite les billes de broyage en vidant la composition sur un filtre.

Un vernis pour enfants au parfum de fraise peut alors être fabriqué en mélangeant à 100 parties du vernis incolore obtenu précédemment, 7,5 parties de la pâte rouge ci-dessus, 0,5 partie de parfum fraise et de l'eau en quantité suffisante pour atteindre une viscosité de 600 à 800 cps à 60 tours/mn au Brookfield mobile 3.

Ce vernis rouge peut s'appliquer en une seule couche, son parfum persiste sur l'ongle après démaquillage. Le séchage est correct et la dureté et l'adhérence de la composition permettent de conserver ce vernis sur l'ongle quelques heures. Sa tenue est suffisante pour permettre un lavage rapide des mains. Pour se démaquiller on trempe les mains dans l'eau et on brosse légèrement.

Un vernis rouge obtenu en mélangeant de la pâte rouge au vernis incolore peut être utilisé pour décorer les ongles. Pour cela, on applique sur les ongles une première couche d'un vernis à ongle classique, on dessine un motif décoratif à l'aide du vernis rouge obtenu selon la présente invention, on recouvre le motif d'une couche de vernis classique, par exemple incolore, pour la protéger lors des lavages de mains.

Cette utilisation est rendue possible par le fait que le vernis selon l'invention se compose d'une émulsion très hydrophile, l'émulsion hydrophile composant le vernis selon l'invention est très résistante aux solvants du type acétates de butyle ou d'éthyle utilisés dans les vernis classiques.

La couche de vernis classique appliquée sur le motif décoratif réalisé en vernis obtenu selon l'invention ne dissout pas ce motif. Une telle réalisation serait impossible en n'utilisant que des vernis à ongles classiques.

Un vernis de soins rouge peut être fabriqué en introduisant dans un récipient muni d'une agitation 100 parties du vernis incolore obtenu précédemment, 0,05 parties de la pâte à teinter rouge, 0,5 parties de parfum de rose, 0,5 partie d'une solution à 50 % dans l'eau de D-Panthénol et la quantité suffisante d'eau pour obtenir une viscosité de 600 à 800 cps à 60 tours/mn au Brookfield mobile 3.

Un tel vernis peut être appliqué le soir, il ne tache pas et est suffisamment résistant pour tenir jusqu'au lendemain et être retiré lors de la toilette.

Il est également possible de proposer un vernis parfumé en ajoutant par exemple 5 parties de parfum violette à 100 parties de vernis incolore. L'ongle s'imprègne du parfum et reste parfumé même après le démaquillage.

Un second exemple concerne la fabrication d'un mascara.

La formule type est :

| Emulsion neutralisée avec une base non volatile 50 à 60 | |
|---|---|
| Phénoxy éthanol ou phénoxy propanol | 0 à 1 |
| Méthyl/Propyl paraben | 0 à 1 |
| TPnB ou coalescent Eb>250°C | 2 à 8 |
| Ether polyoxyéthyléné d'alcool gras | 2 à 8 |
| Emulsion basse Tg | 10 à 30 |
| Hectorite à 10 % dans l'eau | 0 à 10 |
| Epaississant uréthanne | 0 à 0,5 |
| Silicone | 0 à 1 |
| Epaississant acrylique à 33 % dans l'eau | 2 à 10 |
| Parfum | 0 à 0,8 |
| Dispersions pigmentaires | 0 à 10 |
| Eau en quantité suffisante pour régler la viscosité de la composition | |

Les quantités données sont exprimées en pourcentage en poids dans la composition.

Pour fabriquer une pâte teinter noire, on mélange dans un récipient muni d'une agitation rapide 49,5 parties d'eau, 30 parties de Disperbyk 190 (nom commercial d'un agent mouillant commercialisé par Byk Chemie), 0,5 parties de Byk 019 anti mousse (nom commercial de Byk Chemie).

On agite pendant 5 minutes puis on charge sous agitation 20 parties de pigment Noir de carbone.

On introduit ensuite des billes de broyage dans le récipient et on broie pendant 1 heure.

On retire ensuite les billes de broyage en vidant la composition sur un filtre.

Une composition pour mascara peut à titre d'exemple être fabriquée de la manière suivante.

On charge dans un récipient muni d'une agitation 56 parties en poids de Joncryl 8055 (nom commercial d'une émulsion neutralisée avec une base non volatile commercialisée par SC Johnson Polymer).

On prépare une solution en mélangeant 6 parties de EPH (phénoxy éthanol), 44,5 parties de TPnB, 5 parties de Paraben, 1,5 parties de p Paraben et 43 parties de Volpo N3 (nom commercial d'un éther polyoxyéthyléné d'alcool gras commercialisée par Croda).

On ajoute goutte à goutte 10 parties de ce mélange dans le récipient et on agite 15 minutes.

On ajoute ensuite sous agitation 25 parties de Joncryl 8250 Tg=-40°C (nom commercial d'une émulsion à basse Tg commercialisée par SC Johnson Polymer), puis 6,5 parties de Laponite XLS à 10 % dans l'eau (nom commercial d'une hectorite commercialisée par Laporte), 0,3 parties de Byk 346 (nom commercial d'un silicone commercialisé par Byk Chemie), 0,5 parties de Acrysol A44 (nom commercial d'un épaississant uréthanne commercialisé par Rohm & Haas), 0,5 parties de parfum chèvrefeuille, 5 parties de la pâte à teinter noire décrite ci-dessus et 6 parties de Viscoatex 538C 33 % dans l'eau (nom commercial d'un épaississant acrylique commercialisé par Coatex).

Un tel mélange permet d'obtenir une composition ayant une viscosité brookfield mobile 3 de 6000 cps à 6 tours/mn.

Ce mélange s'applique parfaitement sur les cils en donnant un film gainant et brillant qui est parfaitement "waterproof" mais peut être démaquillé avec de l'eau, avec un démaquillant usuel ou avec un démaquillant spécialement conçu pour les mascaras "waterproof". Sa souplesse est suffisante pour éviter la lourdeur des cils.

Ce mascara peut également être proposé en satiné ou mat.

## Revendications

1. Composition cosmétique aqueuse formant par séchage un film démaquillable à l'eau, **caractérisée en ce qu'**elle contient une dispersion de copolymères styrène acrylique fabriquée par polymérisation en émulsion dans l'eau et neutralisation à l'aide d'une base peu volatile, et **en ce que** ladite dispersion a une teneur en solides d'environ 46 % en poids et est présente en quantité telle que le taux de solide dans la composition est compris entre 20 et 40 % en poids.

2. Composition selon la revendication 1, **caractérisée en ce que** l'indice d'acide de ladite dispersion est inférieur à 80 mg de KOH par gramme de polymère, et de préférence inférieur à 50 mg de KOH par gramme de polymère.

3. Composition selon l'une ou l'autre des revendications 1 et 2, **caractérisée en ce que** la dispersion mise en oeuvre est une émulsion de copolymères styrène acrylique ayant une température de transition vitreuse élevée, neutralisée à l'aide d'une base minérale, telle que de la soude ou de la potasse, ou d'une base organique non votatile comme une amine non volatile, triéthanolamine ou tris(hydroxyméthyl)aminométhane.

4. Composition selon l'une quelconque des revendications 1 à 3, **caractérisée en ce que** sa température minimale de formation de film est adaptée à sa destination par l'ajout d'un coalescent et/ou un plastifiant qui favorise la formation du film.

5. Composition selon la revendication 4, **caractérisée en ce que** le coalescent est choisi parmi ceux ayant une température d'ébullition supérieure à 250 °C.

6. Composition selon la revendication 5, **caractérisée en ce que** sa sensibilité à l'eau est adaptée par l'ajout d'un tensioactif du type éther polyoxyéthyléné d'alcool gras.

7. Composition selon l'une quelconque des revendications précédentes, **caractérisée en ce qu'**elle comporte une émulsion présentant une température de transition vitreuse et une température de formation de film très basses.

8. Composition selon l'une quelconque des revendications précédentes, **caractérisée en ce que** sa resolubilisation à la goutte après séchage et coalescence est effectuée en moins de 30 secondes.

9. Composition selon l'une quelconque des revendications précédentes, **caractérisée en ce qu'**elle comprend des additifs tels que des agents de conservation, des produits siliconés, des épaississants minéraux ou organiques, des agents mouillants, des additifs d'amertume, des pigments colorés ou des parfums.

10. Composition selon l'une quelconque des revendications précédentes, **caractérisée en ce qu'**elle est résistante aux solvants du type acétates de butyle et d'éthyle utilisés dans les vernis classiques ce qui permet de l'utiliser pour réaliser des motifs décoratifs sur les ongles, lesdits motifs étant recouverts d'une couche de vernis classique.

## Claims

1. An aqueous cosmetic composition forming by drying a water-removable film, **characterised in that** it contains a dispersion of styrene-acrylic copolymers produced by emulsion polymerisation in water and neutralisation using a sparingly volatile base, and **in that** said dispersion has a solids content of approximately 46% by weight and is present in a quantity such that the amount of solid in the composition is between 20 and 40% by weight.

2. A composition according to Claim 1, **characterised in that** the acid number of said dispersion is less than 80 mg of KOH per gramme of polymer, and preferably less than 50 mg of KOH per gramme of polymer.

3. A composition according to one or the other of Claims 1 and 2, **characterised in that** the dispersion used is an emulsion of styrene-acrylic copolymers having a high glass transition temperature, neutralised using an inorganic base, such as sodium hydroxide or potassium hydroxide, or a non-volatile organic base such as a non-volatile amine, triethanolamine or tris(hydroxymethyl)-aminomethane.

4. A composition according to any one of Claims 1 to 3, **characterised in that** its minimum film-forming temperature is adapted to its intended use by the addition of a coalescent agent and/or a plasticiser which promotes the formation of the film.

5. A composition according to Claim 4, **characterised in that** the coalescent agent is selected from among those having a boiling point higher than 250°C.

6. A composition according to Claim 5, **characterised in that** its sensitivity to water is adapted by the addition of a surfactant of the fatty alcohol polyoxyethylene ether type.

7. A composition according to any one of the preceding claims, **characterised in that** it comprises an emulsion having a very low glass transition temperature and film formation temperature.

8. A composition according to any one of the preceding claims, **characterised in that** its drop resolubilisation after drying and coalescence is effected in less than 30 seconds.

9. A composition according to any one of the preceding claims, **characterised in that** it comprises additives such as preservatives, silicone-containing products, inorganic or organic thickening agents, wetting agents, bitterness additives, coloured pigments or perfumes.

10. A composition according to any one of the preceding claims, **characterised in that** it is resistant to the solvents of the ethyl and butyl acetates type used in conventional varnishes, which makes it possible to use it to produce decorative patterns on finger- and toenails, said patterns being covered with a layer of conventional varnish.

## Patentansprüche

1. Wäßrige kosmetische Zusammensetzung, die durch Trocknen einen mit Wasser abschminkbaren Film bildet, **dadurch gekennzeichnet, daß** sie eine Dispersion von Acrylstyrol-Copolymeren enthält, die durch Polymerisation in einer Emulsion in Wasser und Neutralisation mittels einer gering flüchtigen Base hergestellt wird, und daß die Dispersion einen Feststoffgehalt von etwa 46 Gew.-% aufweist und in einer solchen Menge vorhanden ist, daß der Feststoffgehalt in der Zusammensetzung im Bereich von 20 bis 40 Gew.-% liegt.

2. Zusammensetzung nach Anspruch 1, **dadurch gekennzeichnet, daß** der Säurewert der Dispersion weniger als 80 mg KOH pro Gramm Polymer und vorzugsweise weniger als 50 mg KOH pro Gramm Polymer beträgt.

3. Zusammensetzung nach einem der Ansprüche 1 oder 2, **dadurch gekennzeichnet, daß** die hergestellte Zusammensetzung eine Emulsion von Acrylstyrol-Copolymeren mit einer erhöhten Glasübergangstemperatur ist, die mit einer mineralischen Base, wie z. B. Natriumkarbonat oder Kalium, oder mit einer nicht-flüchtigen, organischen Base, wie z. B. einem nicht-flüchtigen Amin, Triethanolamin oder Tris(hydroxymethyl)aminomethan, neutralisiert wird.

4. Zusammensetzung nach einem der Ansprüche 1 bis 3, **dadurch gekennzeichnet, daß** ihre Mindestfilmbildetemperatur durch Zugabe eines Koaleszenzmittels und/oder eines Weichmachers, das/der die Filmbildung begünstigt, deren Bestimmung entsprechend eingestellt wird.

5. Zusammensetzung nach Anspruch 4, **dadurch gekennzeichnet, daß** das Koaleszenzmittel unter solchen mit einer Siedetemperatur von mehr als 250°C ausgewählt ist.

6. Zusammensetzung nach Anspruch 5, **dadurch gekennzeichnet, daß** ihre Wasserempfindlichkeit durch Zugabe eines Tensids vom Typ Fettalkohol-Polyoxyethylen eingestellt wird.

7. Zusammensetzung nach einem der vorhergehenden Ansprüche, **dadurch gekennzeichnet, daß** sie eine Emulsion mit einer sehr niedrigen Glasübergangstemperatur und Filmbildetemperatur umfaßt.

8. Zusammensetzung nach einem der vorhergehenden Ansprüche, **dadurch gekennzeichnet, daß** ihre Resolubilisierung zu Tropfen nach Trocknung und Koaleszenz in weniger als 30 Sekunden durchgeführt wird.

9. Zusammensetzung nach einem der vorhergehenden Ansprüche, **dadurch gekennzeichnet, daß** sie Additive, wie Konservierungsmittel, silikonhaltige Produkte, mineralische oder organische Verdickungsmittel, Netzmittel, Bitterstoffe, Farbpigmente oder Parfums, umfaßt.

10. Zusammensetzung nach einem der vorhergehenden Anspruche, **dadurch gekennzeichnet, daß** sie beständig gegenüber Lösungsmitteln vom Typ Butyl- und Ethylenacetat ist, die bei den üblichen Lacken verwendet werden, was es gestattet, sie zur Ausbildung von Ziermotiven auf den Nägeln einzusetzen, wobei diese Motive mit einer üblichen Lackschicht überzogen werden.
